# EUROPEAN PATENT APPLICATION

(11) **EP 3 624 132 A1**
(43) Date of publication of application: **18.03.2020**
(21) Application number: 18194295.4
(22) Date of filing: 13.09.2018
(51) Int. Cl.: G16H 30/20, G16H 50/20

(54) **CALCULATING BOUNDARY CONDITIONS FOR VIRTUAL FFR AND IFR CALCULATION BASED ON MYOCARDIAL BLUSH CHARACTERISTICS**

(71) Applicant: Koninklijke Philips N.V., 5656 AG Eindhoven (NL)
(72) Inventor: VAN LAVIEREN, Martijn Anne, 5656 AE Eindhoven (NL); HAASE, Christian, 5656 AE Eindhoven (NL); VAN DER HORST, Arjen, 5656 AE Eindhoven (NL); GRASS, Michael, 5656 AE Eindhoven (NL)
(74) Representative: de Haan, Poul Erik

(57) **Abstract**

An apparatus for assessing a patient's vasculature is provided which is adapted to derive a physiological model of the vasculature including a geometric model and a fluid dynamics model of a vessel of interest and a dynamics measure indicative of a contrast agent dynamics through the vasculature from a plurality of diagnostic images and to analyze the at least one dynamics measure as the function of time such as to adjust at least one boundary condition of the fluid dynamics model.

## Description

### FIELD OF THE INVENTION

The present invention relates to an apparatus for assessing a patient's vasculature, a corresponding method and a respective computer program. In particular, the present invention relates to an apparatus and a method for improving fluid dynamics modeling a patient's vasculature by taking into account the microvascular variations for each patient.

### BACKGROUND OF THE INVENTION

A correct assessment of a coronary disease is of great importance for determining optimized treatment options. This assessment requires knowledge about the hemodynamics in the coronary physiology. To that end, hemodynamic indices such as the Fractional Flow Reserve (FFR) or Instantaneous Wave-Free Ratio (iFR, which is also known as Instantaneous Wave-Free Ratio) may be used to correctly assess coronary artery disease in patients. Both, FFR as well as iFR, are a measure for the pressure drop of the blood along a vessel of interest e.g. due to a stenosis in said vessel of interest. Specifically, they may be determined as the ratio of the pressure distal the stenosis (P_{d}) to the pressure in the aorta (Pₐ).

Hereby, FFR measurements have to be performed during maximal blood flow, i.e. under hyperemia, which may cause discomfort in the patient. In contrast, iFR measurements are performed at rest during a specific period in diastole, thereby avoiding the necessity to induce hyperemia in the patient.

In the past, FFR and/or iFR measurements were typically performed invasively, by measuring the pressure at at least one distal position and one proximal position in the vessel of interest, i.e. at a position distal from and proximate to the aorta. In recent years, approaches have been made to determine the FFR and/or iFR values non-invasively by means of the so-called "virtual" FFR and/or iFR approach. In accordance with the virtual approach, the fluid dynamics in the coronary arteries of a patient are simulated on the basis of a physiological model derived from non-invasively obtained diagnostic image data of the patient's coronary physiology. For this purpose, the physiological model includes a fluid dynamics model representing the blood flow through the coronary vessels.

The fluid dynamic of the blood flow through the vessels is influenced by factors such as vessel wall composition, vessel impedance, vessel wall elasticity or the like. Thus, in order to provide a fluid dynamics model that accurately predicts the blood flow, these factors have to be accounted for in terms of respective boundary conditions. These boundary conditions are generally based on empirical values rather than being determined for each patient. Accordingly, the fluid dynamics model is inherently generalized in that respect, thereby reducing the accuracy of the physiological model of the coronary physiology.

### SUMMARY OF THE INVENTION

It is therefore an object of the present invention to provide an improved apparatus and a corresponding method for modeling the coronary physiology of a patient.

It is a further object of the invention to provide an apparatus and a system for modeling the coronary physiology which allows to determine the boundary conditions for each patient individually. Even more particularly, it is an object of the invention to provide an apparatus and a method which improves the accuracy for image-based virtual assessment of the coronary physiology of a patient by taking into account the variations in microvascular function of the coronary physiology.

This objective is achieved by an apparatus for assessing a patient's vasculature, comprising a processing unit adapted to derive, from at least one diagnostic image of the vasculature, a physiological model of the vasculature, the physiological model comprising a two-dimensional geometric model of a vessel of interest and a fluid dynamics model representing the fluid dynamics through the vessel of interest, to derive, from a time series of diagnostic images of the vasculature, at least one dynamics measure indicative of a contrast agent dynamic through the vasculature as a function of time, to analyze the at least one dynamics measure as the function of time, and to adjust at least one boundary condition of the fluid dynamics model based on the analyzing of the at least one dynamics measure.

One important factor in the assessment of a patient's vasculature and, in particular, of the coronary physiology of a patient are the variations in microvascular function. The impact of these variations may particularly be considered when determining the boundary conditions for patients with diffuse or focal coronary artery disease. A quantitative measure of the microvascular function may hereby be obtained by determining the flow dynamics through the coronary physiology, for example from a time series of images in which the inflow and outflow of a fluid in the vicinity of the vessel of interest is tracked over time. This is enabled by the apparatus as described herein above.

In that context, the term diagnostic image may particularly refer to an image representing the patient's vasculature. Hereby, the term vasculature may refer to a vessel tree or a single vessel. The term vasculature may particularly refer to a vessel segment of the vessel of interest, for which the physiological modeling is performed. In some embodiments, the diagnostic image may represent a vasculature including a vessel of interest of the coronary vasculature.

The at least one diagnostic image may be obtained by a diagnostic imaging modality, such as computed tomography (CT), ultrasound imaging or magnetic resonance imaging.

The diagnostic imaging modality may particularly be gated. Hereby, the gated diagnostic imaging modalities may typically employ a gated reconstruction, in which the acquisition of the diagnostic images is performed in parallel with acquisition of data providing information over the cardiac cycle, such as electrocardiogram (ECG) or photoplethysmographic (PPG) data. This data may hereby be used to gate the image acquisition and the reconstruction by means of respectively selected phase points of the cardiac cycle.

A physiological model of the vessel of interest may be derived from the at least one diagnostic image. For this purpose, the processing unit may particularly be adapted to provide a modeling unit or modeling sub-unit which generates the physiological model based on the at least one diagnostic image. In order to do so, the modeling unit may segment the vessel of interest into a plurality of segments. On the basis of this segmentation, the physiological model of the vessel of interest may be generated, which comprises a geometric model of the vessel of interest, i.e. a geometric representation of the vessel of interest and/or the entire vasculature. This geometric model may typically be a two-dimensional model. In some embodiments, the geometric model may also be a quasi three-dimensional model with the third dimension interpolated from the other two dimensions.

The physiological model may further comprise a fluid dynamics model representing the blood flow through the vessel of interest and/or a blood flow through multiple vessels in the vasculature. The fluid dynamics model may typically be integrated with the two-dimensional geometric model insofar as it represents the fluid dynamics of the blood at each position in the vessel or vessels of interest shown in the geometric model.

In this context, the fluid dynamics model refers to a model of the blood flow through the vessel of interest. This fluid dynamics model is generated by simulating the interaction of the blood with the vessel wall. Since the blood is a fluid and the vessel walls may be considered surfaces with which the fluid interacts, the interaction of the blood with the vessel walls may most accurately be defined by respective boundary conditions that take account of the properties of the vessel wall and the blood interacting with it. These properties may include vessel wall composition, vessel wall elasticity and vessel impedance, bifurcations in the vessel, outflow through these bifurcations, blood viscosity, the vessel outlet resistance at a distal position and the like.

The initial generation of the fluid dynamics model is hereby performed using generalized boundary conditions. The term generalized may hereby refer to the same boundary conditions being used for all patients or to boundary conditions being used for particular patient groups (distinguished by age, gender, physiological condition or the like). These generalized boundary conditions may then be adjusted to improve the accuracy of the fluid dynamics model.

In some embodiments, the deriving may also encompass an importing a physiological model from a prior examination session. In some embodiments, the physiological model may have been previously derived from diagnostic images that have been acquired during a previous session. In this case, the processor does not have to generate the physiological model from diagnostic image data but may simply retrieve the physiological model from a storage for further usage. It shall be understood that the retrieved physiological model may particularly comprise a two-dimensional geometric model and a fluid dynamics model, but may also comprise a three-dimensional geometric model.

Further to the at least one diagnostic image, the apparatus is adapted to receive a time series of diagnostic images. In this context, the term time series of diagnostic images may particularly refer to a plurality of diagnostic images that may have been obtained using the above-cited imaging modalities for a particular measurement time. That is, each diagnostic image of the time series corresponds to a particular point in measurement time. This allows to track dynamic processes, such as the evolution of contrast agent, through the vasculature presented in the diagnostic image.

The time series of diagnostic images is used to derive at least one dynamics measure indicative of the contrast agent dynamic and, as such, the flow dynamic through the vasculature. This dynamics measure is hereby derived as a function of time. This may particularly be achieved by determining a value for the at least one dynamics measure for each diagnostic image of the time series. These values as determined for the dynamics measure may then be analyzed as a function of time. This analysis allows for a better understanding of the contrast agent dynamics and, thus, of the flow dynamics through the vessel of interest and/or the proximate vasculature, thereby allowing to draw conclusions with respect to the microvascular function and/or microvascular resistance of the patient's vasculature.

Based on the analysis, it is possible to adapt the boundary conditions of the fluid dynamics model to account for the patient-specific microvascular properties, such as the specific microvascular function and/or microvascular resistance. In some embodiments, particularly the outflow resistances at bifurcations of the vessel of interest and the outlet resistance at a distal end of the vessel of interest may be derived on the basis of the analysis. Upon adjusting the boundary conditions, a more accurate flow simulation is possible. This allows for a more precise simulation of flow and/or pressure related indices. As an example, the accuracy of virtual iFR and/or virtual FFR may be improved by such an adjustment of the fluid dynamics model.

Alternatively or additionally, the deriving of the dynamics measure may also encompass the retrieval of a dynamics measure that has been obtained previously on the basis of a time series of diagnostic images. That is, the dynamics measure has been determined for the patient during a previous treatment session and stored afterwards. Accordingly, the processor of the apparatus does not have to actively determine the dynamics measure from the time series of diagnostic images, but may be adapted to retrieve said dynamics measure from a storage.

In some embodiments, the at least one diagnostic image and/or the time series of diagnostic images are obtained using X-ray angiography. X-ray angiography is a diagnostic imaging technique particularly well-suited to visualize blood vessels in the (human) body. X-ray angiography is typically performed by injecting a contrast agent into the blood vessels and subsequently irradiating the body part with the contrast agent-filled blood vessels with X-ray radiation to obtain a two-dimensional image in which the contrast agent-filled blood vessels are clearly visible.

The at least one diagnostic image and/or the time series of diagnostic images may thus correspond to at least one X-ray angiography image or a time series of X-ray angiography images representing at least one vessel of interest. More particularly, the at least one diagnostic image from which the physiological model is generated may be a single two-dimensional X-ray angiography image of a vessel of interest.

In some embodiments, the at least one diagnostic image of the vasculature is extracted from the time series of diagnostic images of the vasculature. In some embodiments, the at least one diagnostic image is acquired separately from the time series of diagnostic images. Alternatively, the at least one diagnostic image may be one of the time series of diagnostic images. That is, the at least one diagnostic image used to generate the physiological model may be selected - extracted - from the time series of diagnostic images. In some embodiments, a plurality of diagnostic images may be extracted from the time series for generation of the physiological model. Hereby, the plurality of diagnostic images may particularly have been obtained from the same perspective. Alternatively, diagnostic images from different perspectives may be used.

In any case, it should be considered that, in order to allow to obtain a sufficiently well-resolved physiological model from the at least one diagnostic image or the plurality of diagnostic images, the diagnostic images should hereby have sufficient contrast agent filling. Furthermore, the degree of foreshortening and overlap in the diagnostic images should be sufficiently low. This increases the quality and simplifies the generation of the physiological model.

In some embodiments, the processing unit is adapted to derive the two-dimensional geometric model of the vessel of interest from a single image of the vasculature.

A single diagnostic image may suffice to determine a two-dimensional geometric model of the vessel of interest and a respective fluid dynamics model simulating the fluid dynamics through said vessel of interest. For that purpose, the vessel of interest shown in the single diagnostic image may be segmented and, based on the segmentation, a two-dimensional geometric model of at least one segment of the vessel may be generated. In that context, it is also possible to generate a quasi-three-dimensional geometric model of the at least one vessel segment by interpolating the third dimension, e.g. on the basis of the visible dimensions or on the basis of densitometry data. Further to the geometric model, a fluid dynamics model for simulating the fluid flow through the vessel of interest is generated from the single diagnostic image.

According to some embodiments, the processing unit is adapted to derive the two-dimensional geometric model of the vessel of interest from a plurality of images of the vasculature acquired from a single perspective.

In some embodiments, a plurality of images may be used to obtain the geometric model. In some embodiments, the plurality of images used to obtain the geometric model may correspond to a plurality of images which have all been acquired from the same imaging perspective. Hereby, each of the plurality of images may be registered to a single coordinate system. Using multiple images from the same perspective allows to remove artifacts or to compensate for insufficient contrast, motion blur or vessel overlap at certain locations in the image that would otherwise result in the vessel outline being rendered uncertain or ambiguous.

In some embodiments, the plurality of diagnostic images may particularly correspond to a sequence of diagnostic images of the vessel of interest having sufficient contrast agent filling can be acquired with a low degree of foreshortening and a low overlap. The plurality of diagnostic images may hereby correspond to the entire sequence of diagnostic images or to a selected plurality of diagnostic images. For both cases, the plurality of images may have been acquired from a single perspective or from multiple perspectives.

A vessel segmentation of the vessel of interest in the plurality of images is performed. Based on this segmentation, the physiological model including the geometric model and the fluid dynamics model is generated. In some embodiments, where the plurality of images has been acquired from a single perspective, the segmentation may particularly comprise an averaging step. This results in the segmentation to be more robust. In some embodiments, the processing unit is adapted to derive the at least one dynamics measure by phase-matching each of the diagnostic images of the time series of diagnostic images.

In order to properly analyze the at least one dynamics measure in the vicinity of the vessel of interest and, particularly, in the distal area thereof, with respect to contrast enhancement over time, the diagnostic images of the time series should be phase-matched. The usage of a phase-matched subtraction approach may positively influence the accuracy of the analysis result. The phase-matched subtraction approach may include an image-based registration. In some embodiments, the area that should be analyzed may particularly be determined based on an anatomical atlas. In case of X-ray angiography, the projection angle between the C-arm and the vessel of interest may further be used as prior knowledge, along with the vessel segmentation.

In some embodiments, normalization of the respective values, such as the values of enhancement level of the contrast agent, may be beneficial to achieve more robust results. In this case, the acquisition angle, the contrast agent concentration and the contrast agent injection rate may serve as basis for the normalization. Further, the used X-ray protocol and the patient mass may be used. Alternatively or additionally, a second myocardial reference tissue area can be measured and used for normalization.

According to some embodiments, the fluid dynamics model comprises a lumped parameter model, and wherein the at least one dynamics measure may be used to train the lumped parameter model.

In some embodiments, the fluid dynamics model may comprise or correspond to a lumped parameter model. The term lumped parameter model refers to a model in which the fluid dynamics of the vessels are approximated by a topology of discrete entities. As an example, a vasculature, such as a vessel tree, may be represented by a topology of resistor elements each having a particular resistance. Accordingly, the outlet at a distal end of the vessel is also represented by a particular resistor element. This resistor element is then connected to ground such as to represent the termination of the vessel. Similarly, respective resistor elements may be connected to the series of resistor elements representing the vessel of interest, such as to represent the outflow from the vessel of interest at certain bifurcations. These resistor elements may typically also be connected to ground.

These lumped parameter models reduce the number of dimensions compared to other approaches such as Navier-Stokes or the like. Accordingly, using a lumped parameter model may allow for a simplified calculation of the fluid dynamics inside the vessels and may ultimately result in reduced processing time. The employing of such a lumped parameter model is described for example in international application WO 2016/087396.

In some embodiments, the values determined for the dynamics measure as a function of time may be used to train the lumped parameter model. That is, in some embodiments, the values may have been determined prior to and/or during simulation of the lumped parameter model. The values of the dynamics measure may then be integrated into the training of the lumped parameter model to establish more precise boundary conditions. The training may particularly be performed in a training phase. In such a training phase, the model parameters like the resistance weights or the transfer function parameters that allow to convert the value of the dynamics measure, such as a myocardial blush value, into a virtual outlet resistance value, may be adapted. This adaption may be achieved using a dataset that includes measured reference values. In some embodiments, these measured reference values may for example correspond to known FFR values, iFR, values or myocardial resistance values. The model parameters are hereby adjusted such that the predicted parameters, predicted by the simulation match the measured ground truth.

In some embodiments, the vasculature of the patient comprises a coronary vasculature, and wherein the at least one dynamics measure indicative of the contrast agent dynamic through the vasculature comprises a measure of the myocardial blush.

One particular approach to determine the flow dynamics through the coronary arteries is the video densitometric analysis of the myocardial blush in the tissue of the heart muscle. This analysis may be performed by analyzing a time sequence of image data, such as angiographic image data, in which the vicinity of the vessel of interest is visible. A value for the myocardial blush, for example the mean grey level visible in the respective images, may be derived for each diagnostic image of the time series or for only a subset of the diagnostic images of the time series. Since each diagnostic image corresponds to a particular point in measurement time this allows to determine a quantitative value, such as the mean grey level, for each point in measurement time. The values may then be regarded as a function of measurement time to allow analyzing the contrast agent dynamics during the measurement. As an example, the increase of the mean grey level may show contrast enhancement over time.

The myocardial blush information may thus be used to obtain information about the microvascular function and/or the microvascular resistance. This allows to integrate these aspects into the fluid flow model by tailoring the boundary conditions, such as the outlet resistance of the vessel of interest as well as the outflow resistances of the vessel of interest, in accordance with the determined microvascular function and/or microvascular resistance. Subsequently, a simulation of a hemodynamic index, such as FFR or iFR, may be performed using the updated boundary conditions. This allows to simulate the hemodynamic indices with higher accuracy as the patient-specific microvascular properties are taken into account.

In some embodiments, the at one boundary condition of the fluid dynamics model comprises an outlet resistance at the outlet of the vessel of interest and/or one or more outflow resistances at respective outflows along the vessel of interest. According to some embodiments, the processing unit is further performed to scale a value of the outlet resistance and/or one or more values of the one or more outflow resistances based on the analyzing of the at least one dynamics measure

As indicated herein above, the boundary conditions that are adjusted may, in some embodiments, particularly comprise the outlet resistance at a distal end of the vessel of interest. Alternatively or additionally, the boundary conditions may comprise the outflow resistances at outflow bifurcations of the vessel of interest. That is, the dynamics measure, in particular the myocardial blush, may be analyzed as a function of time and the resulting values may then be used to adapt the outlet resistance as well as the outflow resistances. Hereby, the resistance may particularly be scaled. Based on the analysis. As an example, in case the myocardial blush is regarded, a delayed and/or reduced blush corresponds to a larger resistance). In some embodiments, the values may also be integrated into the fluid dynamics model as indicated herein above. Time-dependent quantitative measures for indicating the dynamics measure may be used. As an example, when considering the myocardial blush, the time to peak, the time to maximum upslope or the like may be used. These time-dependent parameters tend to be more robust, since they avoid the necessity of measuring the total enhancement itself.

In some embodiments, the processing unit is further adapted to derive, based on the fluid dynamics model and the at least one adjusted boundary condition, at least one hemodynamic index, wherein the hemodynamic index is one of a fractional flow reserve and/or an instantaneous Wave-Free Ratio.

The fractional flow reserve (FFR) and the instantaneous Wave-Free Ratio (iFR) are two important hemodynamic indices for assessing coronary artery disease. More specifically, these values allow to determine severity of a stenosis in a vessel of interest. However, FFR and iFR typically need to be measured during an invasive procedure. Hereby, it may often occur that the intravascular measurement results in the FFR and/or iFR value being indicative of a healthy vessel of interest. Thus, many invasive procedures are performed unnecessarily.

In order to avoid this, "virtual" FFR and/or iFR is often used. During the virtual approach, the fluid dynamics model is used to simulate the FFR and/or iFR value. This simulation may particularly be performed by using the updated fluid dynamics model, in which the boundary conditions have been adjusted as described herein above.

In some embodiments the processing unit is further adapted to derive, based on intravascular pressure data, a hemodynamic flow parameter indicative of a blood flow through the vessel of interest.

In some embodiments, the processing unit may further receive intravascular pressure data. Intravascular pressure data may typically be acquired during an intravascular measurement in which a pressure wire is inserted into the vessel of interest. The pressure wire may then be used to obtain a plurality of pressure values at a plurality of intravascular positions along the vessel of interest and provide these values as pressure data to the apparatus. Upon receiving this additional input, the simulation of further hemodynamic parameters, in particular of hemodynamic flow parameters indicative of the flow through the vessel of interest is enabled. For example, the additional provision of intravascular pressure data allows to simulate the flow velocities, the volume flow, coronary flow reserve (CFR) or the like through the vessel of interest.

According to a further aspect method for assessing a patient's vasculature is provided, the method comprising the steps of: deriving, from at least one diagnostic image of the vasculature, a physiological model of the vasculature, the physiological model comprising a two-dimensional geometric model of a vessel of interest and a fluid dynamics model representing the fluid dynamics through the vessel of interest, deriving, from a time series of diagnostic images of the vasculature, at least one dynamics measure indicative of a contrast agent dynamic through the vasculature as a function of time, analyzing the at least one dynamics measure as the function of time and adjusting at least one boundary condition of the fluid dynamics model based on the analyzing of the at least one dynamics measure

In a further aspect, a computer program for controlling an apparatus according to the invention is provided, which, when executed by a processing unit, is adapted to perform the method steps according to the invention. In an even further aspect, a computer-readable medium is provided having stored thereon the above-cited computer program.

It shall be understood that the apparatus of claim 1, the method of claim 13, the computer program of claim 14 and the computer-readable medium of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
FIGURE 1 schematically illustrates an apparatus for assessing a patient's vasculature according to an embodiment.
FIGURE 2 illustrates an example curve for determining a dynamics measure according to an embodiment.
FIGURE 3 illustrates an example fluid dynamics model implemented as a lumped parameter model according to a further embodiment.
FIGURE 4 represents a flow chart for a method for assessing a patient's vasculature according to an embodiment.
FIGURE 5 schematically illustrates an apparatus for assessing a patient's vasculature according to a further embodiment.
FIGURE 6 schematically illustrates an apparatus for assessing a patient's vasculature according to an even further embodiment.

### DETAILED DESCRIPTION OF EMBODIMENTS

The illustration in the drawings is schematically. In different drawings, similar or identical elements are provided with the same reference numerals.

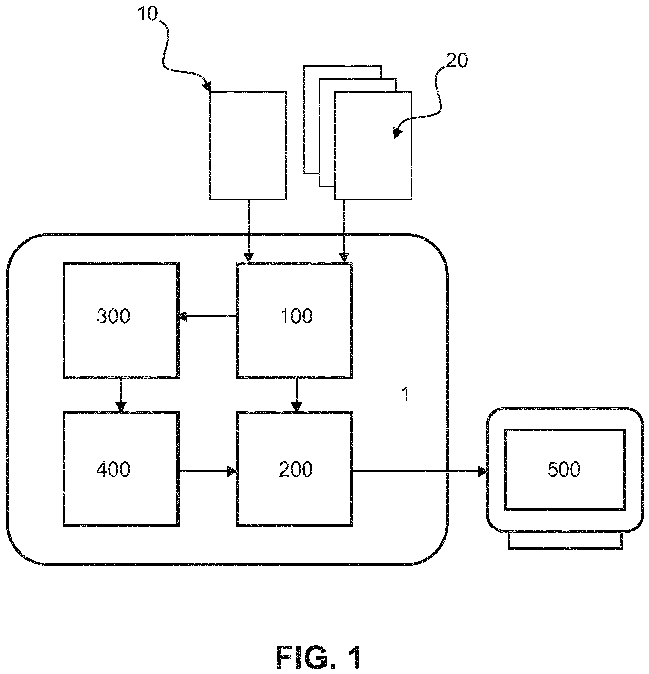

Fig. 1 represents schematically an exemplary embodiment of an apparatus 1 for assessing a patient's vasculature. In the exemplary embodiment of Fig. 1, the apparatus 1 comprises a processing unit comprising one or more processors. In the exemplary embodiment of Fig. 1, the processing unit is sub-divided into an input unit 100, a modelling unit 200, an analyzation unit 300 and a calculation unit 400. Further, the processing unit is communicatively connected to a display unit 500 which comprises, in the exemplary embodiment of Fig. 1, a display screen and, optionally, a user interface. The user interface may hereby be implemented as a physical interface, such as a keyboard or may be provided as a graphical user interface.

In the exemplary embodiment of Fig. 1, the input unit 100 may be adapted to receive a diagnostic image 10 and a time series of diagnostic images 20. In the embodiment, diagnostic image 10 and the diagnostic images 20 of the time series are obtained by X-ray angiography using a contrast agent and are representing the coronary physiology.

In some embodiments, to obtain a diagnostic image 10, an angiography sequence of the contrast agent filled vessel of interest is acquired with a low degree of foreshortening, a low overlap and a sufficient contrast agent filling. Subsequently, a single frame from this sequence is selected which is used as diagnostic image 10 input into apparatus 1. Further, an angiography sequence of approximately 5 to 20 seconds duration of the vessel of interest is acquired which represents the contrast agent enhancement in the myocardium as well as the outflow of the contrast agent through the coronary sinus. In the exemplary embodiment of Fig. 1, this angiography sequence corresponds to the time series of diagnostic images 20 provided to apparatus 1 via input unit 100.

Input unit 100 may then provide the diagnostic image 10 to modeling unit 200. Modelling unit 200 performs a vessel segmentation of the vessel of interest represented in diagnostic image 10. Based on the vessel segmentation, modeling unit 200 generates a physiological model including a two-dimensional geometric model and a fluid dynamics model of the vessel of interest. In some embodiments, the modeling unit may also be adapted to generate a quasi-three-dimensional geometric model of the vessel of interest using a single diagnostic image 10 by interpolating the third dimension based on the visible dimensions, e.g. by assuming a circular cross section of the vessel of interest. In some embodiments the interpolation may also be based on densitometry data.

The fluid dynamics model generated by modeling unit 200 has initial boundary conditions which are based on empirical data. In some embodiments, the initial boundary conditions may also already involve patient-specific boundary conditions.

Input unit 100 may also provide the time series of diagnostic images 20 to analyzation unit 300. Analyzation unit 300 uses the time series of diagnostic images 20 to derive the contrast agent dynamic as a function of time. In the embodiment according to Fig. 1, analyzation unit 300 determines, based on the time series, a dynamics measure indicative of a contrast agent dynamic through the vasculature. More specifically, in the embodiment according to Fig. 1, the analyzation unit 300 derives a myocardial blush value from each of the time series of diagnostic images 20.

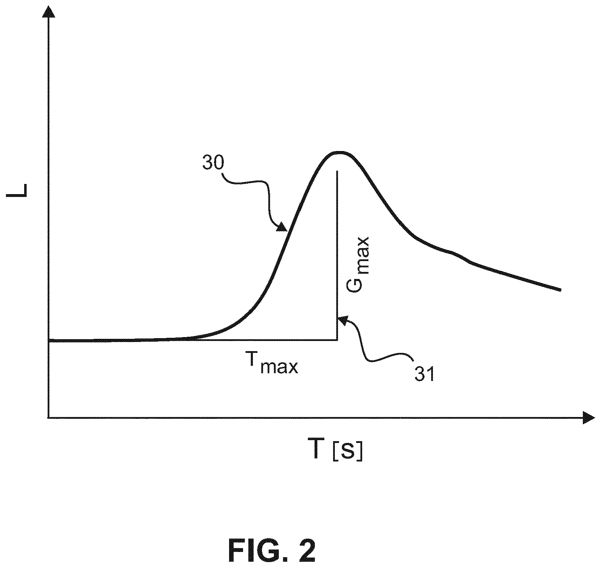

This is done by analyzing the vicinity of the vessel of interest as represented in the time series of diagnostic images 20 with respect to the contrast agent dynamics, particularly the contrast enhancement. In some embodiments, the analysis may be specifically directed at a distal area of the vessel of interest. The analysis may particularly encompass phase-matching the individual diagnostic images in the time series of diagnostic images 20 with one another. In some embodiments, particularly a phase-matched subtraction approach may be used. Prior to subtraction, the individual diagnostic images may be registered to one another, i.e. it may be determined, for each diagnostic image, which position in the target area in one image corresponds to which position in the target area in another image. Subsequently, the contrast enhancement is derived for each diagnostic image of the time series. More specifically, the mean grey level of the area in the vicinity of the vessel of interest is determined for each of a plurality of points in time, whereby the time series of diagnostic images 20 comprises a respective diagnostic image for each point in time of the plurality of points in time. Analyzation unit 300 may plot the contrast enhancement as a function of time as e.g. represented in Fig. 2 as discussed herein below.

The curve representing the evolution of the mean grey level as a function of time may then be normalized in order to achieve more robust results. In some embodiments, the normalization may be performed with respect to the acquisition angle, the contrast agent concentration, the injection rate of the contrast agent, the patient mass, the used X-ray protocol and the like. In other embodiments, a further myocardial reference tissue area may be measured to compensate for these influences.

The analysis result is then provided to computation unit 400. Computation unit 400 may derive, based on the analysis result, respective boundary conditions. In the exemplary embodiment of Fig. 1, computation unit 400 uses the analysis result to derive the outlet resistance as well as the outflow resistances of the vessel of interest. Computation unit may optionally scale the outlet resistance and the outflow resistances based on the determined blush quantity prior to providing them to modeling unit 200, by assuming that a larger resistance is present in the case of reduced or delayed blush and a smaller resistance is present in the case of enhanced blush. In some embodiments, rather than using scaled resistances, modeling unit 200 may directly use the dynamics measure, and in particular the myocardial blush values, for training of the fluid dynamics model.

Modeling unit 200 then adjusts the boundary conditions of the fluid dynamics model in accordance with the (scaled) outlet resistance and/or the outflow resistances, thereby rendering the fluid dynamics model more patient-specific and, thus, more precise.

Fig. 2 illustrates a graphical representation of the analyzing result of analyzation unit 300. The mean grey level L is represented as a function of time T in seconds s as curve 30. In order to obtain further information about the microvascular variations in the patient, curve 30 may be analyzed to determine the time to peak and maximum enhancement 31. For this purpose, the time Tₘₐₓ between the lowest level of contrast enhancement and the reaching of the peak of contrast enhancement is determined. Further in the exemplary embodiment of Fig. 2, the height of said peak, Gₘₐₓ, may be determined. In other embodiments, further values may be used, such as the maximum upslope of the curve 30 or the like.

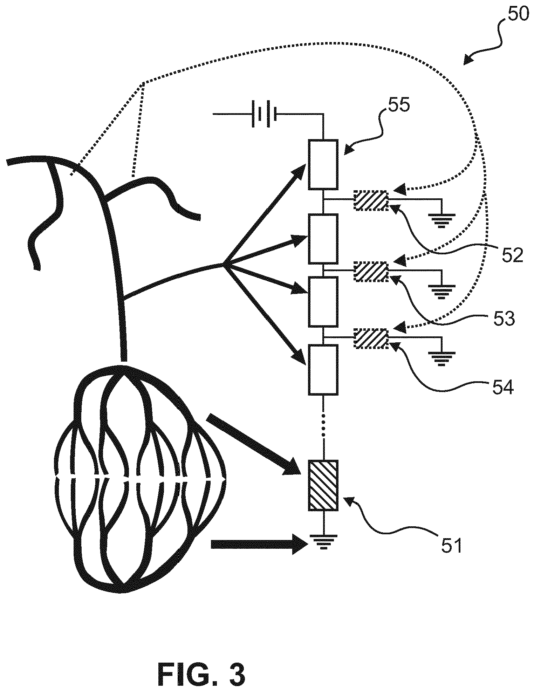

Fig. 3 schematically shows a specific embodiment of a fluid dynamics model 50 of a vessel of interest. In the exemplary embodiment according to Fig. 3, the fluid dynamics model corresponds to a lumped parameter model 50. In the lumped parameter model 50 the fluid dynamics of the vessels are approximated by a topology of discrete (electrical) entities, such as resistor elements and ground elements. In the exemplary embodiment of Fig. 3, the vessel of interest is approximated by a topology 55 of resistor elements each having a particular resistance. The vessel outlet of the vessel of interest is approximated by a resistor 51 and a corresponding ground element. The vessel outflows along the length of the vessel of interest are also each approximated by respective resistor elements 52, 53 and 54 with each being provided with a corresponding ground element. The resistances of resistor elements 51, 52, 53 and 54 are hereby set as boundary conditions of the lumped parameter model. Initially, these resistances may be selected according to empirical values. Upon analysis of the myocardial blush as e.g. described in relation to the exemplary embodiments of Figs. 1 and 2, it is possible to adjust one or more of the resistances 51, 52, 53 and/or 54 in accordance with the analysis to obtain a fluid dynamics model which takes account of the microvascular variation of the specific patient.

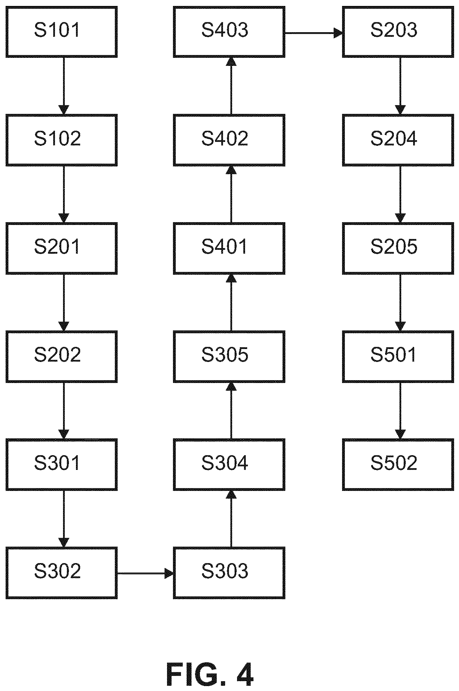

Fig. 4 schematically illustrates a flow chart for a method for assessing a patient's vasculature according to an embodiment. In the exemplary embodiment according to Fig. 4, the processing unit is again sub-divided into an input unit 100, a modeling unit 200, an analyzation unit 300 and a calculation unit 400, respectively.

In step S101, the input unit 100 receives a diagnostic image 10 and a time series of diagnostic images 20, which, in this embodiment, have been acquired of a patient's coronary physiology by X-ray angiography using a contrast agent. In step S102, the input unit 100 provides the diagnostic image 10 to modeling unit 200 and the time series of diagnostic images 20 to analyzation unit 300

In step S201, modeling unit 200 performs a vessel segmentation of the vessel of interest represented in diagnostic image 10. In step S202, modeling unit 200 generates, based on the vessel segmentation, a physiological model including a two-dimensional geometric model and a fluid dynamics model of the vessel of interest having initial boundary conditions which are based on empirical data.

In the exemplary embodiment of Fig. 4, in step S301, analyzation unit 300 uses the time series of diagnostic images 20 to determine a dynamics measure indicative of a contrast agent dynamic through the vasculature by phase-matching the individual diagnostic images in the time series of diagnostic images 20 with one another. In the exemplary embodiment of Fig. 4, the dynamics measure corresponds to the myocardial blush, whereby a blush value is determined for each diagnostic image of the time series of diagnostic images 20. The applied phase-matching may involve a registration of the individual diagnostic images as described herein above.

In step S302, the contrast enhancement is derived for each diagnostic image of the time series. In the exemplary embodiment of Fig. 4 this encompasses that the mean grey level of the area in the vicinity of the vessel of interest is determined for a plurality of points in time, whereby the time series of diagnostic images 20 comprises a respective diagnostic image for each point of the plurality of points in time.

In step S303, analyzation unit 300 may plot the contrast enhancement as a function of time. It shall be understood that this "plotting" may be performed virtually, i.e. that no plot is actually represented to the user. To that end, the plotting may particularly refer to the analyzation unit associating a particular myocardial blush value with a particular point in time, thereby gaining knowledge about the evolution of the mean grey level as a function of time. In step S304, analyzation unit 300 may normalize the mean great level, e.g. with respect to the acquisition angle, the contrast agent concentration, the injection rate of the contrast agent, the patient mass, the used X-ray protocol and the like. In step S305, the analysis result is provided to computation unit 400.

In step S401, computation unit 400 derive respective boundary conditions for the fluid dynamics model based on the analysis result. In the exemplary embodiment of Fig. 4, computation unit 400 derives the outlet resistance as well as the outflow resistances of the vessel of interest.

In step S402, computation unit 400 optionally scales the outlet resistance and the outflow resistances based on the determined myocardial blush quantity and, in step S403, provides the resistance values to modeling unit 200.

In step S203, modeling unit 200 then adjusts the boundary conditions of the fluid dynamics model in accordance with the scaled outlet resistance and the scaled outflow resistances and, in step S204, uses the thus adjusted fluid dynamics model to compute the fluid flow through the vessel of interest. In some embodiments, the fluid dynamics model is particularly used to calculate flow and pressure related hemodynamic indices such as the fractional flow reserve, the instantaneous wave-free ratio, the coronary flow reserve or the like.

In step S205, the modeling unit may provide the physiological model including the two-dimensional geometric model and the fluid dynamics model to display unit 500. In step S501, display unit 500 may generate a graphical representation of the physiological model and, in step S502, present the graphical representation to a user.

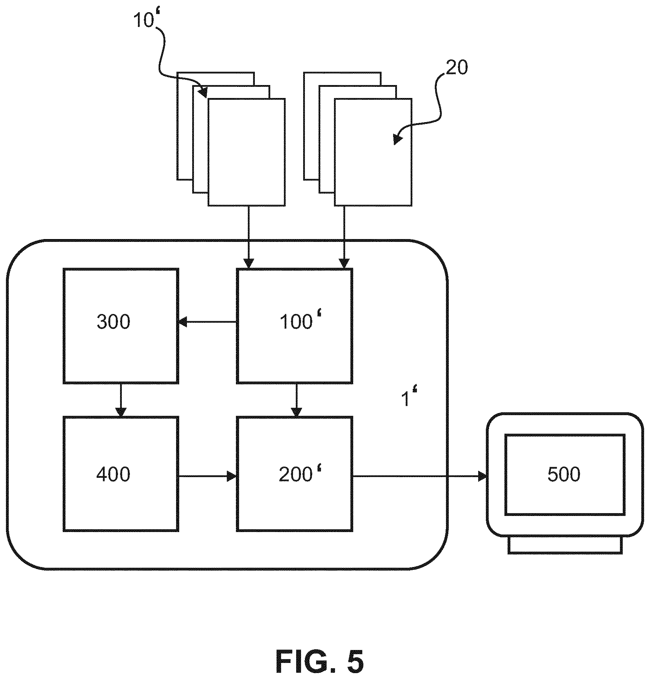

Fig. 5 represents schematically an apparatus 1' for assessing a patient's vasculature according to a further embodiment. The apparatus 1' is largely similar to apparatus 1 as described in relation to Fig. 1. To that end, like elements are provided with like reference numerals. That is, the processing unit of apparatus 1' is also sub-divided into an input unit 100', a modeling unit 200', an analyzation unit 300 and a calculation unit 400, respectively.

The input unit 100' is adapted to receive a time series of diagnostic images 20. Further, the input unit 100' is adapted to receive a plurality - or sequence - of diagnostic images 10'. That is, in contrast to the embodiment according to Fig. 1, the input unit 100' does not receive a single diagnostic image 10, but a sequence of diagnostic images 10'. This sequence may particularly correspond to an X-ray angiographic sequence of the vessel of interest which has been acquired with a low degree of foreshortening, low overlap and sufficient contrast agent filling.

Subsequently, the input unit 100' optionally selects a plurality of diagnostic images from the sequence of diagnostic images 10'. The selected plurality of diagnostic images may particularly correspond to a plurality of diagnostic images, whereby each diagnostic image has been acquired from the same perspective.

Input unit 100' then provides the plurality of diagnostic images selected from the sequence to modeling unit 200'. Modeling unit 200' may then use the plurality of diagnostic images to generate a physiological model of the vessel of interest. That is, modeling unit 200' may be configured to perform a vessel segmentation on the vessel of interest shown in the plurality of images and to generate, based on the vessel segmentation, the respective model.

The further steps performed the embodiment according to Fig. 5 then correspond to those described in relation to Fig. 1. That is, input unit 100' may provide the time series of diagnostic images 20 to analyzation unit 300 and analyzation unit 300 uses the time series of diagnostic images 20 to derive a dynamics measure indicative of a contrast agent dynamic, such as the myocardial blush. In the exemplary embodiment according to Fig. 5, analyzation unit 300 determines a myocardial blush value for each diagnostic image of the time series of diagnostic images 20 as described above and may provide an analysis result including a measure of the myocardial blush (such as the mean grey level of the diagnostic images) as a function of time to calculation unit 400.

Subsequently, calculation unit 400 may derive the respective boundary conditions for the fluid dynamics model comprised in the physiological model. In the exemplary embodiment of Fig. 5, computation unit 400 may particularly derive the outlet resistance and the outflow resistances of the vessel of interest, optionally scale these resistances based on the determined blush quantity and provide them to modeling unit 200' with modeling unit 200' then adjusting the boundary conditions of the fluid dynamics model accordingly.

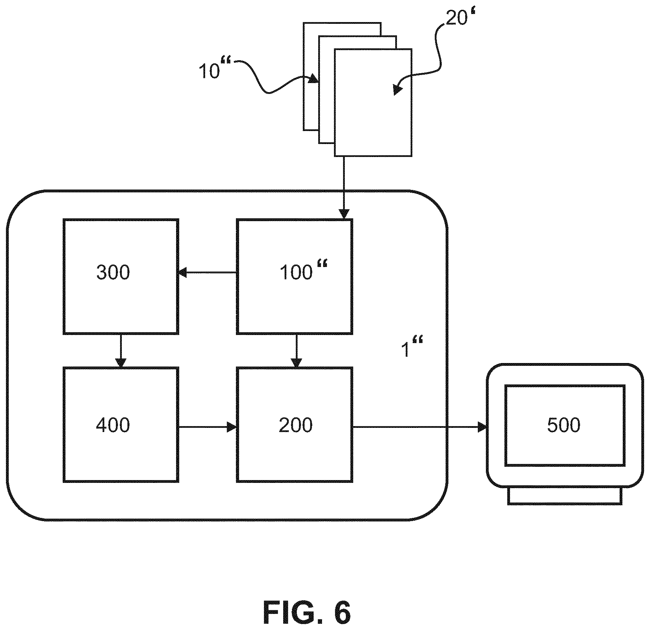

Fig. 6 schematically illustrates an apparatus 1" for assessing a patient's vasculature in accordance with yet another embodiment. Again, the apparatus 1" is largely similar to apparatus 1 as described in relation to Fig. 1 and apparatus 1' as described in relation to Fig. 5 and like elements are provided with like reference numerals.

The processing unit of apparatus 1" is sub-divided into an input unit 100", a modeling unit 200, an analyzation unit 300 and a calculation unit 400, respectively, whereby the input unit 100" is adapted to receive a time series of diagnostic images 20. In contrast to the embodiments according to Figs. 1 and 5, the input unit 100" does not receive any further diagnostic images. Rather, input unit 100" is adapted to select, from the time series of diagnostic images 20, one or more diagnostic images 10" to be provided to the modeling unit 200 for generation of the physiological model. That is, the sequence of diagnostic images 10" used to generate the model is part of the time series of diagnostic images 20 obtained for deriving the dynamics measure which, in the particular embodiment of Fig. 6, corresponds to the myocardial blush. Since the diagnostic images 10" used for modeling shall be acquired with a low degree of foreshortening, low overlap and sufficient contrast agent filling, in some embodiments, the diagnostic images may particularly be selected from diagnostic images of the time series of diagnostic images 20 that have been selected at a later point in time, thereby ensuring that the contrast agent filling is already sufficient. If multiple diagnostic images 10" are selected for the purpose of modeling these multiple images 10" may have been acquired from the same perspective. In some embodiments, the multiple images 10" may also have been acquired from different perspectives.

The one or more diagnostic images 10" may then be provided from input unit 100" to modeling unit 200. Modeling unit 200 may then use the one or more diagnostic images 10" to generate the physiological model which may particularly comprise a two-dimensional geometric model and a fluid dynamics model of the vessel of interest by performing a vessel segmentation of the vessel of interest and then generating the physiological model.

The further steps performed the embodiment according to Fig. 6 then again correspond to those described in relation to Figs. 1 and 5. Thus, input unit 100" may further provide the time series of diagnostic images 20 to analyzation unit 300 and analyzation unit 300 uses the time series of diagnostic images 20 to derive a value for a dynamics measure indicative of a contrast agent dynamic for each diagnostic image of the time series of diagnostic images 20. The analyzation unit 300 may then provide an analysis result including the dynamics measure as a function of time to calculation unit 400. Based on the analysis result, calculation unit 400 may then derive the respective boundary conditions for the fluid dynamics model comprised in the physiological model and provide them to modeling unit 200, which may use these patient-specific boundary conditions to adjust the fluid dynamics model accordingly.

Although in the above-cited embodiments, the diagnostic images have been acquired using X-ray angiography, it shall be understood that other imaging modalities may likewise be used, such as ultra sound imaging, magnetic resonance imaging or the like.

Further, it shall be understood that, while the X-ray imaging has been performed at a single energy, dual energy X-ray measurements may also be used for quantifying the dynamics measure, in particular the myocardial blush, as these measurements allow to more accurately quantify the iodine concentration in the contrast agent. Further, background subtraction is enabled which removes overlaying and disturbing anatomy.

While in the above-cited embodiments, the fractional flow reserve and/or the instantaneous wave-free ratio have been indicated as hemodynamic indices, it shall be understood that further indices may also be derived such as the pressure ratio between the pressure distal to a stenosis and a pressure in the aorta, the coronary flow reserve as well as further, flow-, pressure- or likewise related indices.

Further, while in the above-cited embodiments, the apparatus itself derives the dynamics measure, such as the myocardial blush, from a respective time series of images, it shall be understood that the term deriving is to be interpreted broadly and may also encompass the importing of the measure from computed tomography data, magnetic resonance imaging data, myocardial contrast echography data or the like.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the deriving of the physiological model, the deriving of the at least one dynamics measure, the analyzing of the dynamics measure, the adjusting of the boundary conditions et cetera performed by one or several units or devices can be performed by any other number of units or devices. These procedures in accordance with the invention can hereby be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to an apparatus for assessing a patient's vasculature, comprising a processing unit adapted to derive, from at least one diagnostic image of the vasculature, a physiological model of the vasculature, the physiological model comprising a two-dimensional geometric model of a vessel of interest and a fluid dynamics model representing the fluid dynamics through the vessel of interest, to derive, from a time series of diagnostic images of the vasculature, at least one dynamics measure indicative of a contrast agent dynamic through the vasculature as a function of time, to analyze the at least one dynamics measure as the function of time and to adjust at least one boundary condition of the fluid dynamics model based on the analyzing of the at least one dynamics measure.

## Claims

1. An apparatus for assessing a patient's vasculature, comprising:
a processing unit adapted to
derive, from at least one diagnostic image of the vasculature, a physiological model of the vasculature, the physiological model comprising a two-dimensional geometric model of a vessel of interest and a fluid dynamics model representing the fluid dynamics through the vessel of interest;
derive, from a time series of diagnostic images of the vasculature, at least one dynamics measure indicative of a contrast agent dynamic through the vasculature as a function of time;
analyze the at least one dynamics measure as the function of time, and
adjust at least one boundary condition of the fluid dynamics model based on the analyzing of the at least one dynamics measure.

2. Apparatus according to claim 1, wherein the at least one diagnostic image and/or the time series of diagnostic images are obtained using X-ray angiography.

3. Apparatus according to claim 1, wherein the at least one diagnostic image of the vasculature is extracted from the time series of diagnostic images of the vasculature.

4. Apparatus according to claim 1, wherein the processing unit is adapted to derive the two-dimensional geometric model of the vessel of interest from a single diagnostic image of the vasculature.

5. Apparatus according to claim 1, wherein the processing unit is adapted to derive the two-dimensional geometric model of the vessel of interest from a plurality of diagnostic images of the vasculature acquired from a single perspective.

6. Apparatus according to claim 1, wherein the processing unit is adapted to derive the at least one dynamics measure by phase-matching each of the diagnostic images of the time series of diagnostic images

7. Apparatus according to claim 1, wherein the fluid dynamics model comprises a lumped parameter model, and wherein the at least one microvascular property may be used to train the lumped parameter model.

8. Apparatus according to claim 1, wherein the vasculature of the patient comprises a coronary vasculature, and wherein the at least one dynamics measure indicative of the contrast agent dynamic through the vasculature comprises a measure of the myocardial blush.

9. Apparatus according to claim 1, wherein the at one boundary condition of the fluid dynamics model comprises an outlet resistance at the outlet of the vessel of interest and/or one or more outflow resistances at respective outflows along the vessel of interest.

10. Apparatus according to claim 9, wherein the processing unit is further performed to scale a value of the outlet resistance and/or one or more values of the one or more outflow resistances based on the analyzing of the at least one dynamics measure.

11. Apparatus according to claim 1, wherein the processing unit is further adapted to derive, based on the fluid dynamics model and the at least one adjusted boundary condition, at least one hemodynamic index, wherein the hemodynamic index is one of a fractional flow reserve and/or an instantaneous Wave-Free Ratio.

12. Apparatus according to claim 1, wherein the processing unit is further adapted to derive, based on intravascular pressure data, a hemodynamic flow parameter indicative of a blood flow through the vessel of interest.

13. A method for assessing a patient's vasculature, comprising the steps of:
deriving, from at least one diagnostic image of the vasculature, a physiological model of the vasculature, the physiological model comprising a two-dimensional geometric model of a vessel of interest and a fluid dynamics model representing the fluid dynamics through the vessel of interest;
deriving, from a time series of diagnostic images of the vasculature, at least one dynamics measure indicative of a contrast agent dynamic through the vasculature as a function of time;
analyzing the at least one dynamics measure as the function of time; and
adjusting at least one boundary condition of the fluid dynamics model based on the analyzing of the at least one dynamics measure.

14. A computer program for controlling an apparatus according to anyone of claims 1 to 12, which, when executed by a processing unit, is adapted to perform the method according to claim 13.

15. A computer-readable medium having stored thereon the computer program according to claim 14.
